# EUROPÄISCHE PATENTSCHRIFT

(11) **EP 3 165 886 B1**
(45) Veröffentlichungstag und Bekanntmachung des Hinweises auf die Patenterteilung: **15.07.2020**
(21) Anmeldenummer: 16187352.6
(22) Anmeldetag: 06.09.2016
(51) Int. Cl.: G01G 19/50

(54) **KOMBINATIONSMESSGERÄT ZUR MESSUNG DES GEWICHTS UND MINDESTENS EINES WEITEREN KÖRPERPARAMETERS EINES PROBANDEN**
COMBINATION MEASURING DEVICE FOR MEASURING THE WEIGHT AND AT LEAST ONE OTHER BODY PARAMETER OF A TEST SUBJECT
APPAREIL DE MESURE COMBINÉ DESTINÉ A MESURER LE POIDS ET AU MOINS UN AUTRE PARAMÈTRE PHYSIOLOGIQUE D'UN SUJET

(30) Priorität: 03.11.2015 DE 102015118770
(43) Veröffentlichungstag der Anmeldung: 10.05.2017
(73) Patentinhaber: seca gmbh & co. kg, 22089 Hamburg (DE)
(72) Erfinder: Schmidt, Jan, 22941 Bargteheide (DE)
(74) Vertreter: Uexküll & Stolberg

(56) Entgegenhaltungen:
- EP-A2- 2 565 598
- US-A1- 2011 077 536
- US-A1- 2011 226 035

## Beschreibung

Die Erfindung betrifft ein Kombinationsmessgerät zur Messung des Gewichts und mindestens eines weiteren Körperparameters eines Probanden, das eine Waage mit einer Plattform, die auf wenigstens eine Wägezelle einwirkt, einen Messaufbau zur Messung eines weiteren Körperparameters und eine Datenaufnahme- und -verarbeitungseinrichtung aufweist, die dazu eingerichtet ist, die Messung der Waage und des Messaufbaus zu steuern und für das auf der Plattform lastende Gewicht repräsentative Messsignale der Waage über eine Messperiode als Funktion der Zeit und die Messsignale des Messaufbaus über die Messperiode aufzunehmen, zu verarbeiten und Messergebnisse auszugeben sowie aus Messsignalen der Waage vorab oder zu Beginn der Messperiode ein Gewichtsmessergebnis zu bestimmen und über die Messperiode eine Abweichung der Messignale der Waage von dem Gewichtsmessergebnis zu überwachen.

Es gibt eine Reihe von Kombinationsmessgeräten, die neben einer Waage einen Messaufbau zur Messung eines oder mehrerer weiterer Körperparameter des Probanden umfassen. Ein typisches Beispiel sind Geräte zur Bestimmung von Körperzusammensetzungsparametern (Body Composition Analyzer), die eine Waage und eine Bioimpedanzmessvorrichtung aufweisen, mit der die Impedanz des Körpers und einzelner Körpersegmente eines Menschen gemessen werden können. Die Leitfähigkeit des menschlichen Körpers hängt empfindlich vom Wasseranteil ab. Da die fettfreien Anteile des Körpers wie Muskeln und Körperflüssigkeiten einen Großteil des körpereigenen Wassers beinhalten, während Fettgewebe aufgrund hydrophober Eigenschaften einen sehr geringen Wasseranteil enthält, können aus der Bestimmung der Leitfähigkeit des Körpers oder einzelner Körpersegmente (oder umgekehrt des Widerstands oder der Impedanz) Rückschlüsse auf die relativen Anteile an Fett gezogen werden. Bei gleichzeitiger Messung des Gewichts des Probanden können dann dessen Fettmasse und die fettfreie Masse des Körpers des Probanden und weitere Körperzusammensetzungsparameter wie Wassermasse etc. bestimmt werden.

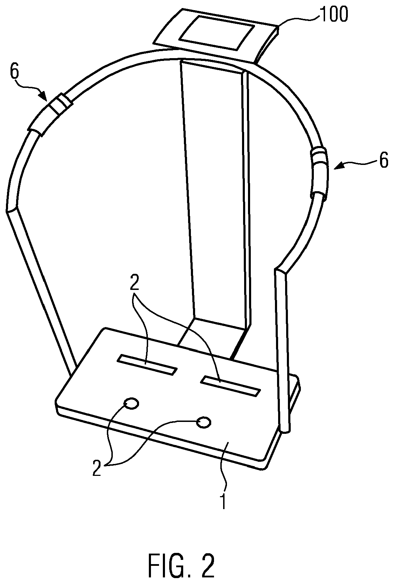

Eine beispielhafte Vorrichtung dieser Art, die zur Bestimmung von Körperzusammensetzungsparametern durch Bioimpedanzanalyse eingerichtet ist, ist in DE 10 2010 023 122 A1 beschrieben. Ein solches Kombinationsmessgerät zur Bestimmung von Körperzusammensetzungsparametern ist auch in Fig. 2 schematisch dargestellt. Die Vorrichtung hat eine über dem Boden gelagerte Plattform 1 einer Waage, die auf darunter liegende Wägezellen einwirkt, um die auf die Plattform 1 einwirkende Gewichtskraft bestimmen zu können. Auf der Plattform befinden sich vier Fußelektroden 2, wobei der Proband mit jedem Fuß auf jeweils zwei der Fußelektroden tritt. Ferner sind an einem herumgeführten Handgriff vier Handelektroden 6 vorgesehen, die die zu untersuchende Person mit den Händen umgreifen muss, so dass jede Hand mit zwei Elektroden Kontakt hat. Dann wird ein Wechselstrom über jeweils zwei an unterschiedlichen Gliedmaßen anliegende Elektroden eingeprägt und an zwei, ebenfalls an unterschiedlichen Gliedmaßen anliegenden Elektroden wird die Spannung gemessen. Durch Übergang zu anderen Paaren von stromeinprägenden Elektroden und spannungserfassenden Elektroden können sukzessive verschiedene Messprogramme durchgeführt und verschiedene Körpersegmente auf ihre Impedanz hin untersucht werden. Die Waage und die Elektroden sind über Leitungen mit einer Datenaufnahme- und -verarbeitungseinrichtung 100 verbunden, die die Messsignale auswertet und Messergebnisse ausgibt.

Bei einem solchen Kombinationsmessgerät mit Waage und Messaufbau zur Bioimpedanzmessung ist es wichtig, dass der Proband sich während der Ausführung der Messprogramme zur Bioimpedanzmessung nicht bewegt, da Bewegungen die Impedanzmessungen verfälschen können.

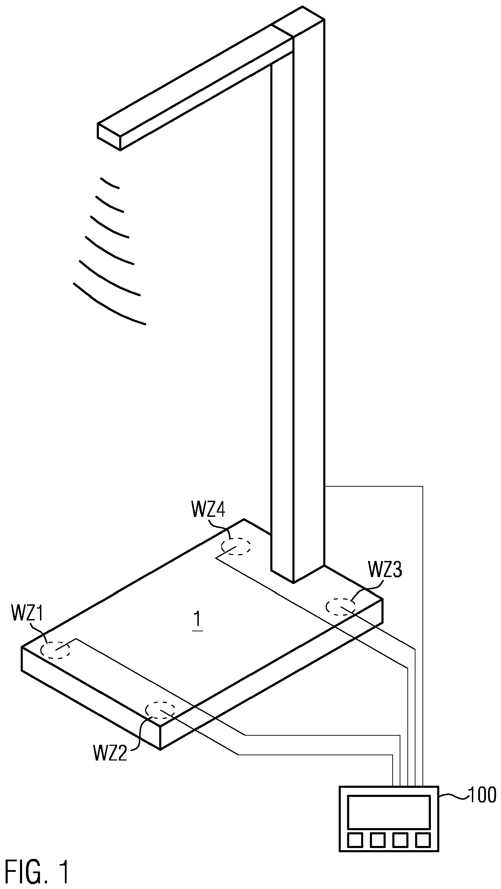

Fig. 1 zeigt schematisch ein weiteres Kombinationsmessgerät. Dieses weist neben einer Waage einen Aufbau zur Messung der Körperlänge auf. Die Waage hat eine Plattform 1, wobei vier darunter liegende Wägezellen WZ1, ... WZ4 schematisch dargestellt sind. Der Messaufbau zur Messung der Körperlänge umfasst einen vertikalen Träger, an dessen oberen Ende ein Querträger befestigt ist. In dem Querträger ist an dem vom vertikalen Träger entfernten Ende ein Ultraschallwandler angeordnet. Der Ultraschallwandler ist so angeordnet, dass bei Anregung abgestrahlter Ultraschallwellen auf die Oberseite des Kopfes eines auf der Plattform der Waage stehenden Probanden gerichtet werden. Die Waage und der Ultraschallwandler sind über Leitungen mit einer Datenaufnahme- und - verarbeitungseinrichtung 100 verbunden, die die Messsignale auswertet. Die Datenaufnahme- und -verarbeitungseinrichtung 100 ist dazu eingerichtet, mit dem Ultraschallwandler eine Laufzeitmessung des an der Oberseite des Kopfes des Probanden reflektierten Ultraschalls durchzuführen. Aus der Laufzeit ergibt sich der Abstand zur Oberseite des Kopfes und damit bei bekannter Höhe des Querträgers die Körperlänge des Probanden, die von der Datenaufnahme- und verarbeitungseinrichtung 100 neben dem Messergebnis der Waage ausgegeben wird. Auch bei einem solchen Kombinationsmessgerät ist es wichtig, dass sich der Proband während der Messperiode, während der die Körperlänge gemessen wird, nicht bewegt, um zu einer genauen und zuverlässigen Messung der Körperlänge zu kommen.

Es gibt eine Reihe weiterer Kombinationsmessgeräte, die neben einer Waage einen weiteren Messaufbau umfassen. Ein weiterer Messaufbau in Kombination mit einer Waage kann zum Beispiel eine Blutdruckmessvorrichtung sein. Ein anderer Messaufbau, der in Kombination mit einer Waage eingesetzt werden kann, ist eine Vorrichtung zur optischen Körpervermessung mit Oberflächenerfassung des Körpers des Probanden. Eine solche Einrichtung zur optischen Körpervermessung kann zum Beispiel ein Laserscanner, eine Stereokameraeinrichtung oder eine TOF-Kamera sein. Es kann in einem Kombinationsmessgerät auch eine Waage mit mehreren Messaufbauten zur Messung verschiedener weiterer Körperzusammensetzungsparameter kombiniert sein.

US 2011/0077536 A1 beschreibt ein Kombinationsmessgerät mit den Merkmalen des Oberbegriffs von Patentanspruch 1. Das Kombinationsmessgerät kann eine Personenwaage mit einem Messaufbau zur Bioimpedanzmessung sein. Um Täuschungsversuche zurückzuweisen, wird vorgeschlagen, die Gewichtsmesssignale auf Abweichungen hin zu überwachen, um die Zuverlässigkeit der Gewichtsmessung zu bewerten, oder das Impedanzmesssignal über die Messperiode zu bewerten, um die Zuverlässigkeit der Bioimpedanzmessung zu überprüfen, oder das Bioimpedanzmesssignal zu überwachen, um die Zuverlässigkeit der Gewichtsmessung zu bewerten. Die Bewertung findet statt, indem die Varianz der Signale über eine Meßperiode bestimmt wird und eine minimale Varianz als Konsistenzkriterium verlangt wird, andernfalls ergibt die Bewertung, dass ein Täuschungsversuch durch einen der Messung unterzogenen Gegenstand vorliegt.

US 2011/0226035 A1 betrifft ein Kombinationsmessgerät mit Personenwaage und Messaufbau zur Bioimpedanzmessung zur Bestimmung der Körperzusammensetzung. Zur Nullpunktdetektion der Waage wird zu Beginn, solange die Messplattform der Waage noch unbelastet ist, eine Reihe von Anfangswerten der Waagesignale aufgezeichnet. Aus diesen Anfangswerten wird dann, wenn deren Varianz eine vorgegebene Schwelle nicht überschreitet, durch Mittelwertbildung ein Anfangsreferenzwert generiert, der bei anschließenden Messungen als Nullpunkt verwendet wird. Die Aufnahme einer Reihe von Anfangswerten im unbelasteten Zustand der Waage kann im Verlauf der Zeit wiederholt werden, um so den detektierten Nullpunkt bei zeitlich veränderlichen Bedingungen zu aktualisieren.

EP 2 565 598 A2 betrifft ein Kombinationsmessgerät mit Personenwaage und einem Messaufbau zur Bioimpedanzmessung. Das Kombinationsmessgerät ist dazu ausgestaltet, das Ergebnis der Bioimpedanzmessung in seinem zeitlichen Verlauf über die Messperiode zur Anzeige zu bringen. Das soll die Person nach Art einer Rückkopplung oder eines Biofeedback in die Lage versetzen, Einfluss auf die Bioimpedanzmessung verändernde Körperfunktionen zu nehmen. Dabei kann vorgesehen sein, dass der Person vorgegeben wird, dass die Bioimpedanzmessung oder der daraus resultierende Körperzusammensetzungsparameter innerhalb eines vorgegebenen Bereichs verbleiben soll.

Bei fast allen dieser Kombinationsmessgeräte treten Fehler in den Messwerten des oder der weiteren Körperparameter auf, wenn sich der Patient während der Messperiode, die für die Erfassung der Daten zur Bestimmung des weiteren Körperparameters benötigt wird, bewegt. Eine Überprüfung auf Bewegung des Probanden findet jedoch, wenn überhaupt, nur indirekt und damit nicht zuverlässig statt. So können instabile Messwerte für den weiteren Körperparameter oder Werte außerhalb eines gültigen Bereichs auf eine fehlerhafte Messung hinweisen. Es gibt aber neben Bewegungen des Probanden viele weitere Ursachen für mögliche Fehler, so dass, wenn nur eine Fehlermeldung ausgegeben wird, der Proband keine Rückmeldung über die Ursache des Fehlers erhält.

Es ist Aufgabe der vorliegenden Erfindung, ein Kombinationsmessgerät mit einer Waage und einem Messaufbau zur Messung eines weiteren Körperparameters so auszugestalten, dass Fehler in dem Messergebnis des weiteren Messaufbaus, die durch Bewegung des Probanden während der Messung verursacht werden, leichter erkennbar sind.

Zur Lösung dieser Aufgabe dient ein Kombinationsmessgerät mit den Merkmalen von Patentanspruch 1. Vorteilhafte Ausführungsformen der Erfindung sind in den Unteransprüchen aufgeführt.

Erfindungsgemäß ist die Datenaufnahme- und -verarbeitungseinrichtung dazu eingerichtet, vor Beginn der Messperiode für den Messaufbau oder zu Beginn der Messperiode ein Gewichtsmessergebnis zu bestimmen und im weiteren Verlauf der Messperiode eine Abweichung des aktuell aufgenommenen Messsignals der Waage von dem Gewichtsmessergebnis zu überwachen. Falls die Abweichung einen vorgegebenen ersten Schwellenwert überschreitet, wird eine auf eine mögliche Bewegung des Probanden hinweisende Verdachtsmeldung erzeugt, um bei der Prüfung der Zuverlässigkeit des Messergebnis für den weiteren Körperparameter verwendet zu werden. Ferner ist die Datenaufnahme- und - verarbeitungseinrichtung dazu eingerichtet, falls die Abweichung der Messsignale der Waage vom Gewichtsmessergebnis einen vorgegebenen zweiten Schwellenwert, der größer als der erste ist, überschreitet, eine auf eine Bewegung des Probanden hinweisende Fehlermeldung zu erzeugen. Bei Überschreiten des größeren zweiten Schwellenwertes kann mit hoher Wahrscheinlichkeit davon ausgegangen werden, dass der Proband eine messungsverfälschende Bewegung durchgeführt hat. Darüber hinaus ist die Aufnahme- oder -verarbeitungseinrichtung dazu eingerichtet, falls die Signale des Beschleunigungssensors einen vorgegebenen zweiten Beschleunigungsschwellenwert überschreitet, der größer als der erste ist, eine auf eine Bewegung des Probanden hinweisende Fehlermeldung zu erzeugen und bei Vorliegen einer Bewegungsfehlermeldung die Messperiode des Messaufbaus abzubrechen und eine auf die Bewegungsfehlermeldung hinweisende Ausgabe zu erzeugen. Wenn eine Bewegungsfehlermeldung vorliegt, ist wie erwähnt mit hoher Wahrscheinlichkeit davon auszugehen, dass eine messverfälschende Bewegung des Probanden stattgefunden hat. In diesem Fall kann die Messung dann unmittelbar abgebrochen werden und der Proband über den Bewegungsfehler informiert werden.
Gemäß der Erfindung ist das Kombinationsmessgerät also mit seiner Datenaufnahme- und -verarbeitungseinrichtung dazu eingerichtet, die ohnehin vorhandenen Messsignale der wenigstens einen Wägezelle der Waage auch nach Bestimmung des Gewichtsmessergebnis weiter zu analysieren, um dadurch während des weiteren Verlaufs der Messperiode für den Messaufbau zur Messung des weiteren Körperparameters mögliche Bewegungen des Probanden zu erfassen, um die Information bei der Überprüfung der Zuverlässigkeit der Messung des weiteren Körperparameters heranzuziehen.

Grundsätzlich können auch herkömmliche Kombinationsmessgeräte mit Waage durch Software- oder Firmware-Update so nachgerüstet werden, dass sie die Funktionen des erfindungsgemäßen Kombinationsmessgerätes mit der Datenaufnahme- und -verarbeitungseinrichtung realisieren können.

In einer vorteilhaften Ausführungsform ist die Datenaufnahme- und -verarbeitungseinrichtung dazu eingerichtet, die Messergebnisse des Messaufbaus über die Messperiode auf vorgegebene Konsistenzkriterien hin zu überprüfen. Eine solche Konsistenzüberprüfung ist zum Beispiel für Bioimpedanzmessungen in WO 2014/117758 A1 beschrieben. Bei Feststellung einer Verletzung eines Konsistenzkriteriums und bei Vorliegen der Bewegungsverdachtsmeldung wird die Messung des Messaufbaus als fehlerhaft verworfen und eine auf die Verdachtsmeldung über die Bewegung des Probanden hinweisende Ausgabe erzeugt. Auf diese Weise erhält der Proband den Hinweis, dass die Messung des weiteren Körperparameters fehlerhaft verlaufen ist, wobei die zusätzliche Angabe des Hinweises auf die Bewegung den Probanden über die wahrscheinliche Ursache der fehlerhaften Messung informiert, so dass er sich in der nächsten Messperiode besser darauf konzentrieren kann, Bewegungen zu vermeiden.

In einer bevorzugten Ausführungsform ist ein Beschleunigungssensor an der Plattform aufgehängt und die Datenaufnahme- und verarbeitungseinrichtung ist dazu eingerichtet, eine auf eine mögliche Bewegung des Probanden hinweisende Verdachtsmeldung zu erzeugen, wenn das Signal des Beschleunigungssensors einen ersten Beschleunigungsschwellenwert überschreitet.

Die Datenaufnahme- und -verarbeitungseinrichtung kann weiter dazu eingerichtet sein, bei vorliegender Bewegungsfehlermeldung und nach Ausgabe des Hinweises darauf eine neue Messperiode für den Messaufbau zur Messung des weiteren Körperparameters zu starten.

In einer bevorzugten Ausführungsform sind unter der Plattform mehrere Wägezellen vorgesehen. Die Datenaufnahme- und -verarbeitungseinrichtung ist dazu eingerichtet, neben der Summe der Messsignale der Wägezellen für jede Wägezelle separat ein Wägezellengewichtsmessergebnis aufzunehmen und über die Messperiode für jede Wägezelle die Abweichung des Messsignals von dem jeweiligen Wägezellengewichtsmessergebnis zu überwachen und, falls die Überwachung eine vorgegebenen Schwellenwert für eine Einzelwägezelle überschreitet, die auf eine Bewegung des Probanden hinweisende Fehlermeldung zu erzeugen. Es gibt Bewegungsarten, z.B. Gewichtsverlagerungen, die sich nicht auf Summe der Wägezellengewichtsmessergebnise (das Gesamtgewichtsmessergebnis bleibt bei einer Gewichtsverlagerungen konstant) auswirken, so dass die gesonderte Überwachung der einzelnen Wägezellen die Sensibilität zur Erfassung von Bewegungen erhöht.

In dieser Ausführungsform mit mehreren Wägezellen ist die Datenaufnahme- und -verarbeitungseinrichtung vorzugsweise weiter dazu eingerichtet, die Differenzen zwischen Wägezellengewichtsmessergebnissen der einzelnen Wägezellen zu bilden und eine auf eine Fehlpositionierung des Probanden hinweisende Fehlpositionierungsverdachtsmeldung zu erzeugen, falls eine Differenz einen vorgegebenen Differenzschwellenwert überschreitet. Dies kann zum Beispiel geschehen, wenn der Proband schräg oder versetzt zu einer Seite auf der Plattform der Waage steht. In der Folge einer solchen fehlerhaften Positionierung kann auch die Messung des Messaufbaus für den weiteren Körperparameter von der Fehlpositionierung betroffen sein. Eine solche Fehlpositionierungsverdachtsmeldung kann bei der Überprüfung der Zuverlässigkeit der Messung des Messaufbaus für den weiteren Körperparameter in die Beurteilung mit einbezogen werden.

Dazu können die Datenaufnahme- und -verarbeitungseinrichtung dazu eingerichtet sein, die Messergebnisse des Messaufbaus über die Messperiode auf vorgegebene Konsistenzkriterien hin zu überprüfen. Falls die Verletzung eines Konsistenzkriteriums festgestellt wird, wird die Messung des weiteren Körperparameters wie üblich verworfen. Im vorliegenden Fall kann darüber hinaus aber ein Hinweis auf die Fehlpositionierung ausgegeben werden, die wahrscheinlich Ursache der fehlerhaften Messung war. Daraufhin kann der Proband vor Start der nächsten Messperiode seine Positionierung überprüfen und gegebenenfalls korrigieren.

Die Erfindung wird im Folgenden im Zusammenhang mit den Zeichnungen näher beschrieben, in denen:
Fig. 1 eine schematische perspektivische Ansicht eines Kombinationsmessgerätes zeigt, das eine Waage und einen Messaufbau zur Messung der Körperlänge umfasst,
Fig. 2 eine schematische perspektivische Ansicht eines Kombinationsmessgerätes mit einer Waage und einem Messaufbau zur Bioimpedanzanalyse zeigt,
Fig. 3 zeigt schematische Graphen der Messsignale von vier Wägezellen WZ1 bis WZ4 als Funktion der Zeit über einen Messperiode (beliebige Einheiten an der Ordinate und Abszisse) sowie einen Graphen für die Summe der Messsignale der Wägezellen, die dem Gesamtgewichtssignal der Waage entspricht,
Fig. 4 zeigt Graphen wie in Fig. 3 für einen anderen Messvorgang,
Fig. 5 zeigt Graphen wie in Fig. 3 und 4 für einen weiteren Messvorgang, und
Fig. 6 zeigt Graphen wie in den Fig. 3 bis 5 für einen weiteren Messvorgang, und
Fig. 7 ein Flussdiagramm der Ablaufsteuerung der Durchführung der Messungen in einem Kombinationsmessgerät in einer Ausführungsform der Erfindung zeigt.

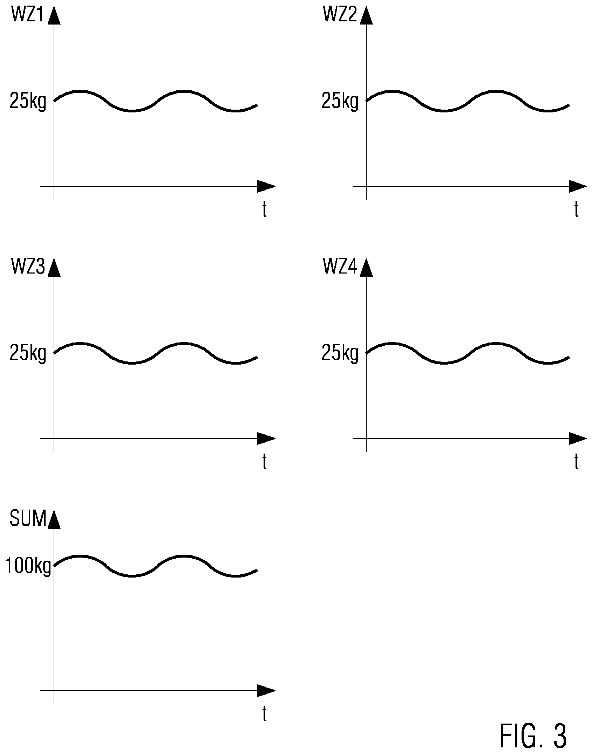

Es ist zu beachten, dass in den Fig. 3 bis 6 der Graph der Summe der Messsignale jeweils im Vergleich zu den anderen vier Graphen der einzelnen Wägezellen eine anders skalierte Y-Achse hat.

Im Folgenden werden zunächst mehrere Messvorgänge mit und ohne Bewegungen des Probanden anhand der schematischen Graphen in Fig. 3 bis 6 erläutert.

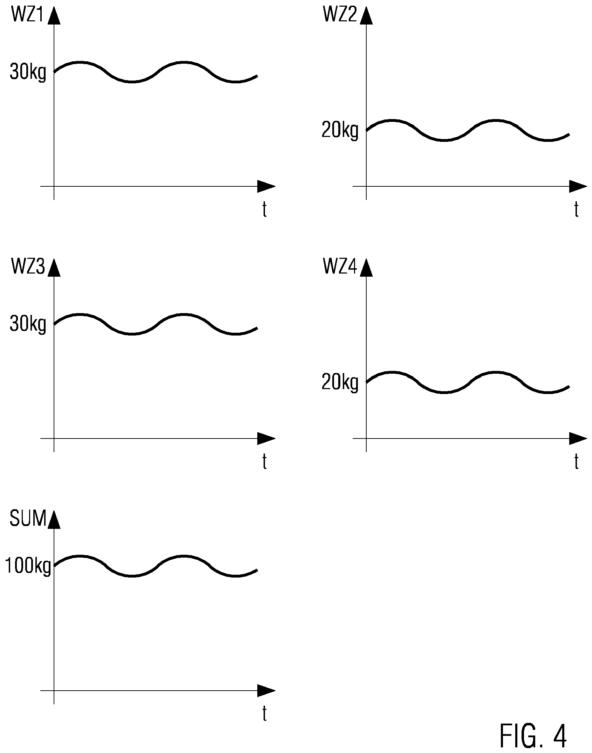

Fig. 3 zeigt einen Messvorgang über eine Messperiode, in der der Messaufbau zur Messung des weiteren Körperparameters die Messung durchführt, wobei der Proband ruhig und mittig auf der Plattform der Waage steht, z.B. auf der Waage des in Fig. 1 gezeigten Kombinationsmessgerätes. Wenn der Proband wie in diesem Fall ruhig und mittig auf der Plattform der Waage steht liefern alle vier Wägezellen bis auf minimale individuelle Abweichungen das gleiche Wägezellensignal. Die Wägezellensignale der Wägezellen WZ1 bis WZ4 sind aber wie angedeutet über die Zeit der Messperiode nicht vollständig konstant, sondern überlagert von kleinen Störungen, die durch Atmung, Herzschlag und gegebenenfalls äußere Einwirkungen verursacht werden. Es ist zu beachten, dass der Graph in Fig. 3 die natürlichen Schwankungen aus Darstellungsgründen mit übertriebener Amplitude zeigt. Tatsächlich sind die natürlichen Schwankungen, die toleriert werden sollen, prozentual geringer. Dies gilt auch für die Graphen in den Figuren 4-6.

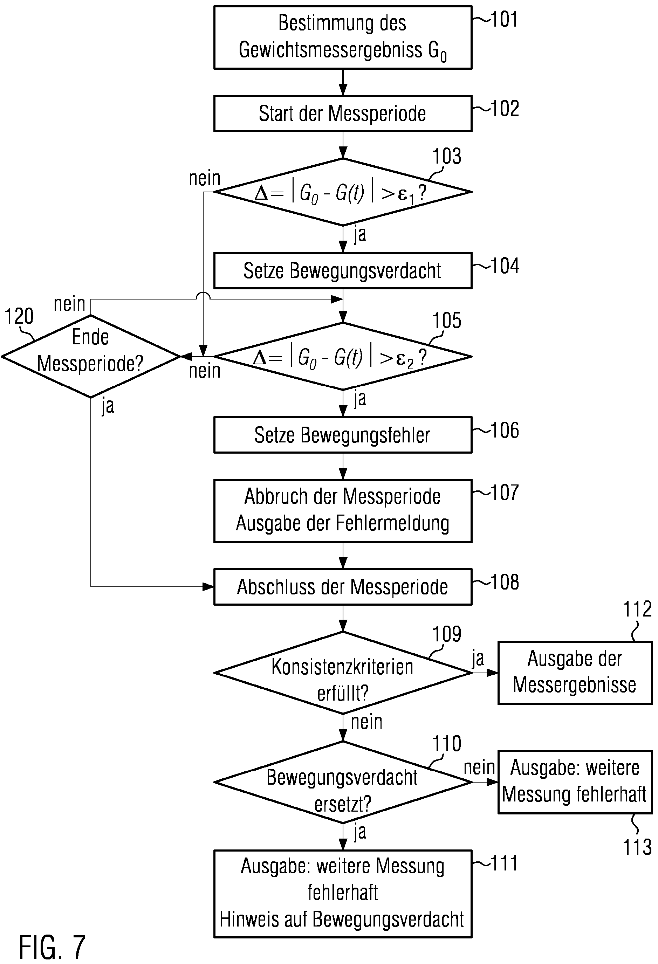

Die Messsignale der vier Wägezellen werden jeweils mit einem Analog-Digital-Wandler umgesetzt und dann die Summe der Wägezellensignale gebildet, die dem Gesamtgewicht entspricht. Vor Beginn der Messperiode oder zu deren Anfang wird das Gesamtgewichtssignal der Waage analysiert und ein Gesamtgewichtsmessergebnis gespeichert, sobald ein Hold-Kriterium erfüllt ist. Dies ist der Fall, wenn das Messsignal über einen vorgegebenen Zeitraum innerhalb eines vorbestimmten Toleranzbandes um den Mittelwert liegt. Nach Bestimmung des Gesamtgewichtsmessergebnisses wird über den Verlauf der Messperiode für die Messung des weiteren Körperparameters der Betrag der Differenz aus Gesamtgewichtsmessergebnis und Gewichtsmesssignal der Waage überwacht. Die bei der Überwachung angewendeten Kriterien werden beispielhaft weiter unten im Zusammenhang mit Fig. 7 beschrieben.

Bei dem in Fig. 3 dargestellten Messvorgang zeigt das Gewichtsmesssignal der Waage, das aus der Summe der vier Wägezellensignale gebildet ist, über die Messperiode nur geringe Schwankungen, die "natürliche" Ursachen haben, wie Puls, Atmung und dergleichen. In diesem Fall überschreitet der Betrag der Differenz des Gesamtgewichtsmessergebnis und des Messsignals der Waage im Verlaufe der Messperiode zu keinem Zeitpunkt den ersten oder sogar den zweiten Schwellenwert. Es wird daher kein Indikator für Bewegungsverdacht und kein Indikator für Bewegungsfehler gesetzt.

Falls also kein Indikator für Bewegungsverdacht und kein Indikator für Bewegungsfehler gesetzt ist und dann bei einer Prüfung von Konsistenzkriterien der Messsignale des weiteren Messaufbaus, kein Kriterium als verletzt gefunden wird, werden das Gesamtgewichtsmessergebnis und Messergebnisse der Messung des Messaufbaus für den weiteren Körperparameter ausgegeben. Falls jedoch eines der Konsistenzkriterien als verletzt gefunden wird, kann nur das Gesamtgewichtsmessergebnis und die Angabe, dass die Messung des weiteren Körperparameters fehlerhaft ist, ausgegeben werden, da kein Hinweis auf Bewegung als mögliche Ursache gefunden wurde.

In Fig. 4 sind die Messsignale der Wägezellen für einen Messvorgang dargestellt, bei dem der Proband diagonal auf der Plattform der Waage steht und die Wägezellen WZ1 (vorne links) und WZ3 (hinten rechts) stärker als die anderen Wägezellen belastet. Obwohl hier die Summe der Messsignale der Wägezellen, die im unteren Graphen als Messsignal der Waage dargestellt ist, von leichten Schwankungen abgesehen statisch ist und insofern wie zuvor kein Indikator für Bewegungsverdacht und kein Indikator für Bewegungsfehler gesetzt wird, kann hier durch Überprüfen von Differenzen der Messsignale der Wägezellen bei Überschreiten eines Differenzschwellenwertes eine Fehlpositionierungverdachtsmeldung erzeugt werden. Diese kann dann ähnlich wie die Bewegungsverdachtsmeldung bei Feststellen der Verletzung eines Konsistenzkriteriums bei der Konsistenzprüfung des Messsignals des Messaufbaus dazu verwendet werden, um neben dem Hinweis, dass das Messergebnis des Messaufbaus fehlerhaft ist, eine Anzeige zum Fehlpositionierungsverdacht auszugeben, um dem Probanden einen Hinweis auf die mögliche Ursache des Fehlers zu geben.

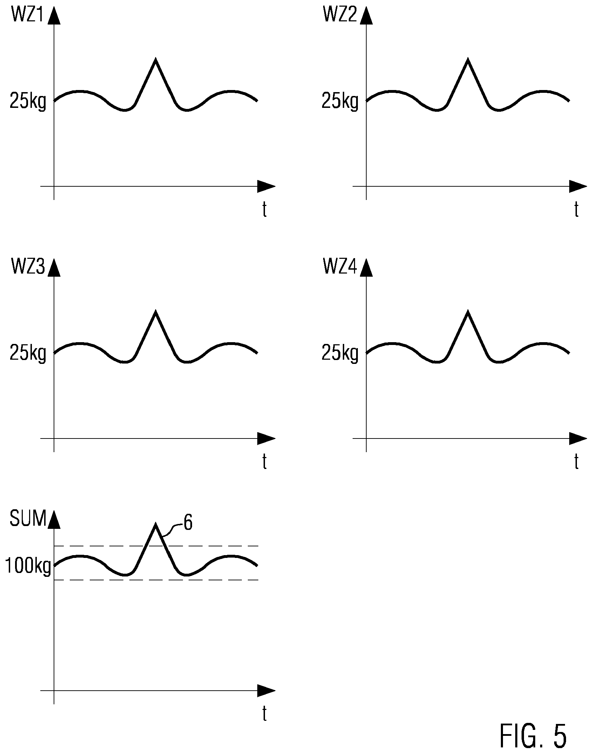

In dem Messvorgang, dessen Graphen in Fig. 5 gezeigt sind, kommt es etwa in der Mitte der Messperiode zu einer kurzen ruckartigen Bewegung des Probanden. Im unteren Graphen in Fig. 5 für das Gesamtgewichtssignal der Waage ist ein Fehlerband mit dem zweiten Schwellenwert eingezeichnet. Verlässt das Gesamtgewichtssignal der Waage dieses Fehlerband, wie in dem Messvorgang von Fig. 5 gegeben, wird ein Indikator für einen Bewegungsfehler gesetzt. Bei der Ausgabe, dass die weitere Messung des Messaufbaus fehlerhaft ist wird, darüber hinaus ein Hinweis auf eine zu starke Bewegung des Probanden ausgegeben, so dass dieser sich in der nächsten Messperiode auf das Vermeiden von Bewegungen konzentrieren kann.

Eine solche ruckartige Bewegung führt z.B. zu einer Verfälschung der Bioimpedanzmessung, wenn der Messaufbau für den weiteren Körperparameter eine Bioimpedanzmessvorrichtung ist. Im Stand der Technik konnten solche gestörten Bioimpedanzmessungen zwar unter Umständen durch Konsistenzprüfungen erkannt werden, dann jedoch nur ausgegeben werden, dass die Bioimpedanzmessung fehlerhaft ist, ohne dass dem Probanden ein Hinweis darauf gegeben werden könnte, dass die Ursache des Fehlers eine zu starke Bewegung war.

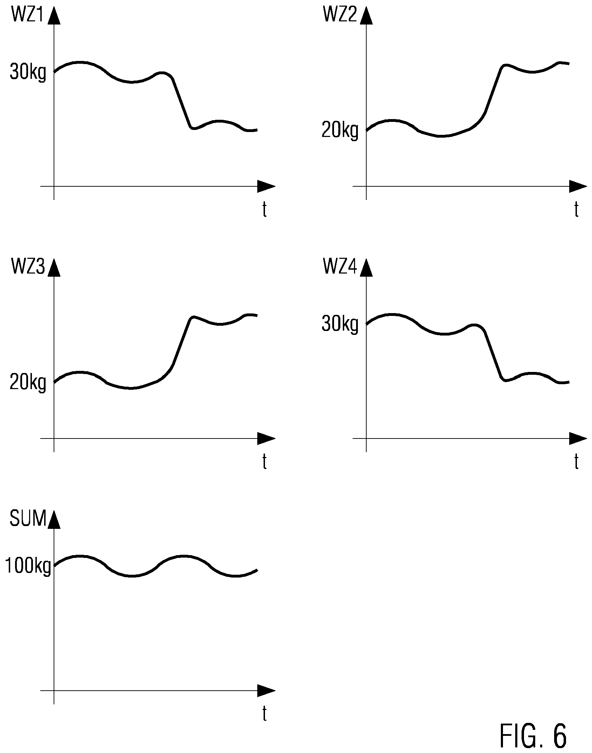

In Fig. 6 sind die Graphen der Wägezellen und des Gesamtgewichtsmesssignals der Waage für einen Messvorgang dargestellt, bei dem der Proband eine langsame, nicht ruckartige Gewichtsverlagerung vornimmt. Die Summe der Einzelsignale der Wägezellen im Gesamtgewichtssignal der Waage im unteren Graphen ist hier zeitlich weitgehend konstant, so dass aufgrund dessen nicht auf eine Bewegung geschlossen werden könnte. Gemäß der vorliegenden Erfindung können jedoch auch die Messsignale der einzelnen Wägezellen dahingehend überprüft werden, ob über die Messperiode die Differenz aus einem Wägezellengewichtsmessergebnis (das für die einzelne Wägezelle wieder vorab oder zu Beginn der Messperiode bestimmt wird) und dem Messignal der Wägezelle im Verlauf der Messperiode dem Betrag nach eine vorgegebene Schwelle für eine Einzelwägezelle überschreitet. Wenn dies der Fall ist, wird eine auf eine Bewegung des Probanden hinweisende Fehlermeldung erzeugt. Diese wird zusammen mit der Meldung, dass die Messung des Messaufbaus für den weiteren Körperparameter fehlerhaft ist, angezeigt, so dass der Proband über die Fehlerursache informiert ist und diese in der nächsten Messperiode vermeiden kann.

In Fig. 7 ist ein Flussdiagramm dargestellt, das die Arbeitsweise der Datenaufnahme- und -verarbeitungseinrichtung nach einer Ausführungsform für das Kombinationsmessgerät zeigt. In diesem Ausführungsbeispiel beginnt der Messvorgang, indem die Datenaufnahme- und -verarbeitungseinrichtung das Gesamtgewichtssignal der Waage analysiert und daraus vorab ein Gesamtgewichtsmessergebnis bestimmt (Block 101). Anschließend wird in Block 102 die Messperiode für den Messaufbau gestartet, wobei in diesem Flussdiagramm keine weiteren Einzelheiten zu den Schritten des Messvorgangs des Messaufbaus für den weiteren Körperzusammensetzungsparameter gezeigt sind.

Nach Start der Messperiode wird in dem Entscheidungsblock 103 überprüft, ob der Betrag der Differenz des Gesamtgewichtsmessergebnisses G₀ und des momentanen Gesamtgewichtssignals G(t) der Waage größer als ein erster Schwellenwert ε₁ ist. Wenn dies nicht der Fall ist, wird im Entscheidungsblock 120 geprüft, ob die Messperiode wegen Zeitablauf zu beenden ist. Wenn dies nicht der Fall ist wird die Überprüfung im Entscheidungsblock 103 wiederholt, bis die Zeit der Messperiode abgelaufen ist, worauf hin der Prozess aus dem Entscheidungsblock 120 zum Abschluss der Messperiode in Block 108 übergeht.

Wenn während der Messperiode in dem Entscheidungsblock 103 ein Überschreiten des ersten Schwellenwertes ε₁ festgestellt wird, wird in Block 104 ein Indikator für Bewegungsverdacht gesetzt. Anschließend wird in Entscheidungsblock 105 geprüft, ob die Abweichung des Gesamtgewichtsmessergebnisses G₀ von den aktuellen Messsignalen G(t) sogar einen zweiten, größeren Schwellenwert ε₂ überschreitet. Wenn ja, wird in Block 106 ein Indikator für Bewegungsfehler gesetzt. Daraufhin wird in Block 107 die Messperiode für den Messaufbau abgebrochen und angezeigt, dass die Messung des Messaufbaus misslungen ist und dass als Fehlerursache eine zu starke Bewegung des Probanden festgestellt worden ist. Danach kann in bestimmten Ausführungsformen automatisch der Start einer weiteren Messperiode in Block 102 erfolgen.

Wird in Entscheidungsblock 105 keine Überschreitung des zweiten Schwellenwertes ε₂ festgestellt, geht der Prozess zum Entscheidungsblock 120 über, wo überprüft wird, ob die Zeit der Meßperiode bereits abgelaufen ist. Wenn ja läuft der Prozess in Block 108 mit dem Abschluss der Meßperiode weiter. Ist die Meßperiode noch nicht zu Ende, geht der Prozess wieder zum Eingang des Entscheidungsblocks 105 über.

Im Entscheidungsblock 109 werden dann die in der Messperiode aufgenommenen Ergebnisse des Messaufbaus auf Konsistenzkriterien hin überprüft. Wenn die Konsistenzkriterien erfüllt sind, werden die Messergebnisse des Messaufbaus in Block 112 ausgegeben. Diese Ausgabe kann natürlich zusammen mit der Ausgabe des Gesamtgewichtsmessergebnisses erfolgen.

Ist eines der Konsistenzkriterien nicht erfüllt wird in Entscheidungsblock 110 geprüft, ob der Indikator für Bewegungsverdacht gesetzt ist. Wenn nicht, kann in Block 113 nur ausgegeben werden, dass die weitere Messung fehlerhaft ist, aber keine mögliche Ursache dafür.

Wenn der Indikator für Bewegungsverdacht gesetzt ist, wird in Block 111 neben dem Hinweis auf die fehlerhafte Messung des Messaufbaus ein Hinweis auf den Bewegungsverdacht ausgegeben.

## Patentansprüche

1. Kombinationsmessgerät zur Messung des Gewichts und eines weiteren Körperparameters eines Probanden, das eine Waage mit einer Plattform (1), die auf wenigstens eine Wägezelle (WZ1, WZ2, WZ3, WZ4) einwirkt, einen Messaufbau zur Messung des weiteren Körperparameters und eine Datenaufnahme- und -verarbeitungseinrichtung (100) aufweist, die dazu eingerichtet ist, die Messung der Waage und des Messaufbaus zu steuern und für das auf der Plattform lastende Gewicht repräsentative Messsignale der Waage über eine Messperiode als Funktion der Zeit und die Messsignale des Messaufbaus über die Messperiode aufzunehmen, zu verarbeiten und Messergebnisse auszugeben, und
aus Messsignalen der Waage vorab oder zu Beginn der Messperiode ein Gewichtsmessergebnis zu bestimmen und über die Messperiode eine Abweichung der Messignale der Waage von dem Gewichtsmessergebnis zu überwachen,
**dadurch gekennzeichnet, dass** die Datenaufnahme- und - verarbeitungseinrichtung (100) ferner dazu eingerichtet ist,
(1.) falls die Abweichung einen vorgegebenen ersten Schwellenwert überschreitet, eine auf eine mögliche Bewegung des Probanden hinweisende Verdachtsmeldung zu erzeugen, um bei der Prüfung der Zuverlässigkeit des Messergebnis für den weiteren Körperparameter verwendet zu werden;
(2.) falls die Abweichung der Messignale der Waage vom Gewichtsmessergebnis einen vorgegebenen zweiten Schwellenwert, der größer als der erste ist, überschreitet, eine auf eine Bewegung des Probanden hinweisende Fehlermeldung zu erzeugen; und bei Vorliegen einer Bewegungsfehlermeldung die Messperiode des Messaufbaus abzubrechen und eine auf die Bewegungsfehlermeldung hinweisende Ausgabe zu erzeugen.

2. Kombinationsmessgerät nach Anspruch 1, **dadurch gekennzeichnet, dass** ein Beschleunigungssensor mit der Plattform verbunden ist und dass die Datenaufnahme- und -verarbeitungseinrichtung (100) dazu eingerichtet ist, eine auf eine mögliche Bewegung des Probanden hinweisende Verdachtsmeldung zu erzeugen, wenn das Signal des Beschleunigungssensors einen ersten Beschleunigungsschwellenwert überschreitet.

3. Kombinationsmessgerät nach Anspruch 1 oder 2, **dadurch gekennzeichnet, dass** die Datenaufnahme- und -verarbeitungseinrichtung (100) dazu eingerichtet ist, die Messergebnisse des Messaufbaus über die Messperiode auf vorgegebene Konsistenzkriterien hin zu überprüfen und bei Verletzung eines Konsistenzkriteriums und Vorliegen der Bewegungsverdachtsmeldung die Messung des Messaufbaus als fehlerhaft zu verwerfen und eine auf die Verdachtsmeldung über die Bewegung des Probanden hinweisende Ausgabe zu erzeugen.

4. Kombinationsmessgerät nach einem der vorhergehenden Ansprüche 2 bis 3, **dadurch gekennzeichnet, dass** die Datenaufnahme- und -verarbeitungseinrichtung (100) dazu eingerichtet ist, falls die Signale des Beschleunigungssensors einen vorgegebenen zweiten Beschleunigungsschwellenwert, der größer als der erste ist, überschreitet, eine auf eine Bewegung des Probanden hinweisende Fehlermeldung zu erzeugen.

5. Kombinationsmessgerät nach Anspruch 4, **dadurch gekennzeichnet, dass** die Datenaufnahme- und -verarbeitungseinrichtung (100) dazu eingerichtet ist, bei Vorliegen der Bewegungsfehlermeldung und nach Ausgabe des Hinweises darauf eine neue Messperiode für den Messaufbau zur Messung des weiteren Körperparameters zu starten.

6. Kombinationsmessgerät nach einem der vorhergehenden Ansprüche, **dadurch gekennzeichnet, dass** eine Mehrzahl von Wägezellen (WZ1, WZ2, WZ3, WZ4) vorgesehen ist und die Datenaufnahme- und -verarbeitungseinrichtung (100) dazu eingerichtet ist, neben der Summe der Messsignale der Wägezellen für jede Wägezelle separat ein Wägezellengewichtsmessergebnis aufzunehmen und über die Messperiode für jede Wägezelle die Abweichung des Messignals von dem Wägezellengewichtsmessergebnis zu überwachen und, falls die Abweichung eine vorgegebenen Schwelle für eine Einzelwägezelle überschreitet, die auf eine Bewegung des Probanden hinweisende Fehlermeldung zu erzeugen.

7. Kombinationsmessgerät nach Anspruch 6, **dadurch gekennzeichnet, dass** die Datenaufnahme- und -verarbeitungseinrichtung (100) dazu eingerichtet ist, die Differenzen zwischen Wägezellengewichtsmessergebnissen der einzelnen Wägezellen zu bilden und, falls eine Differenz einen vorgegebenen Differenzschwellenwert überschreitet, eine auf eine Fehlpositionierung des Probanden hinweisende Fehlpositionierungsverdachtsmeldung zu erzeugen, um bei der Prüfung der Zuverlässigkeit des Messergebnis für den weiteren Körperparameter verwendet zu werden.

8. Kombinationsmessgerät nach Anspruch 7, **dadurch gekennzeichnet, dass** die Datenaufnahme- und -verarbeitungseinrichtung (100) dazu eingerichtet ist, die Messergebnisse des Messaufbaus über die Messperiode auf vorgegebene Konsistenzkriterien hin zu überprüfen und bei Verletzung eines Konsistenzkriteriums und Vorliegen der Fehlpositionierungsverdachtsmeldung die Messung des Messaufbaus als fehlerhaft zu verwerfen, eine dahingehende Ausgabe zu erzeugen und eine auf die Verdachtsmeldung über die Fehlpositionierung des Probanden hinweisende Ausgabe zu erzeugen.

## Claims

1. Combination measuring device for measuring the weight and a further body parameter of a subject, which combination measuring device comprises a weighing scale having a platform (1) which acts on at least one load cell (WZ1, WZ2, WZ3, WZ4), a measuring apparatus for measuring the further body parameter, and a data acquisition and processing unit (100) which is adapted to control the measurement of the weighing scale and of the measuring apparatus, and which is arranged to acquire from the weighing scale measuring signals representing a weight weighing on the platform as a function of time over a measuring period, to process said signals, and to output measurement results, and
to determine a weight measuring result from the measuring signals of the weighing scale in advance or at the beginning of the measuring period, and to monitor over the measuring period a deviation of the measuring signals of the weighing scale from the weight measuring result,
**characterized in that** the data acquisition and processing unit (100) is further adapted
(1.) in case the deviation exceeds a predetermined first threshold value, to generate a suspicion message indicative of a possible movement of the subject, which movement suspicion message is to be used in an examination of the reliability of the measuring result for the further body parameter,
(2.) in case the deviation of the measuring signals of the weighing scale from the weight measuring result exceeds a predetermined second threshold value which is higher than the first threshold value, to generate an error message indicative of movement of the subject; and, in case a movement error message is present, to terminate the measuring period of the measuring apparatus and to generate an output indicative of the movement error message.

2. Combination measuring device according to claim 1, **characterized in that** an acceleration sensor is connected to the platform, and **in that** the data acquisition and processing unit (100) is arranged to generate a suspicion message indicative of possible movement of the subject in case the signal of the acceleration sensor exceeds a first acceleration threshold value.

3. Combination measuring device according to claim 1 or 2, **characterized in that** the data acquisition and processing unit (100) is arranged to check the measuring results of the measuring apparatus over the measuring period for predetermined consistency criteria, and, in case a consistency criteria is found to be violated and a movement suspicion message is present, to discard the measurement of the measuring apparatus as faulty and to generate an output indicative of the suspicion message of movement of the subject.

4. Combination measuring device according to any of the preceding claims 2 to 3, **characterized in that** the data acquisition and processing unit (100) is arranged, in case the signals of the acceleration sensor exceed a second predetermined acceleration threshold value which is higher than the first acceleration threshold value, to generate an error message indicative of a movement of the subject.

5. Combination measuring device according to claim 4, **characterized in that** the data acquisition and processing unit (100) is arranged to, in case a movement error message is present and after an output indicative thereof has been made, to start a new measuring period for the measuring apparatus for measuring the further body parameter.

6. Combination measuring device according to any of the preceding claims, **characterized in that** a plurality of load cells (WZ1, WZ2, WZ3, WZ4) is provided, and that the data acquisition and processing unit (100) is arranged to acquire, in addition to the sum of the measuring signals of the load cells, for each load cell individually a load cell weight measuring result, and to monitor over the measuring period for each load cell the deviation of the load cell measuring signal from the load cell weight measurement result and to generate, in case the deviation exceeds a predetermined threshold for a single load cell, the error message indicative of movement of the subject.

7. Combination measuring device according to claim 6, **characterized in that** the data acquisition and processing unit (100) is arranged to form differences between the load cell weight measurement results and the measuring signals of the individual load cells, and, in case a difference exceeds a predetermined difference threshold value, to generate an incorrect positioning suspicion message indicative of an incorrect positioning of the subject, which incorrect positioning suspicion message is to be used in the examination of the reliability of the measuring result for the further body parameter.

8. Combination measuring device according to claim 7, **characterized in that** the data acquisition and processing unit (100) is arranged to check the measuring results of the measuring apparatus over the measuring period for predetermined consistency criteria, and, in case a consistency criterion as found violated and an incorrect positioning suspicion message is present, to discard the measuring of the measuring apparatus, to generate an output indicative of the faulty measurement, and to generate an output indicative of the incorrect positioning suspicion message.

## Revendications

1. Appareil de mesure combiné pour mesurer le poids et un autre paramètre corporel d'un sujet, qui présente une balance avec une plate-forme (1) qui agit sur au moins une cellule de pesage (WZ1, WZ2, WZ3, WZ4), un montage de mesure pour mesurer l'autre paramètre corporel et un dispositif d'enregistrement et de traitement de données (100), qui est organisé pour commander la mesure de la balance et du montage de mesure et pour enregistrer, traiter, des signaux de mesure de la balance représentatifs du poids chargeant la plate-forme pendant une période de mesure en fonction du temps et les signaux de mesure du montage de mesure pendant la période de mesure, et pour fournir des résultats, et pour déterminer un résultat de mesure de poids à partir de signaux de mesure de la balance préalablement ou au début de la période de mesure et pour surveiller, pendant la période de mesure, un écart des signaux de mesure de la balance par rapport au résultat de mesure de poids, **caractérisé en ce que** le dispositif d'enregistrement et de traitement de données (100) est en outre organisé,
(1.) dans le cas où l'écart excéderait une première valeur de seuil prédéfinie, pour produire un message de soupçon signalant un mouvement possible du sujet, afin d'être utilisé lors du contrôle de la fiabilité du résultat de mesure pour l'autre paramètre corporel ;
(2.) dans le cas où l'écart des signaux de mesure de la balance par rapport au résultat de mesure de poids excéderait une seconde valeur de seuil prédéfinie, qui est supérieure à la première, pour produire un message d'erreur signalant un mouvement du sujet ; et, en présence d'un message d'erreur de mouvement, pour interrompre la période de mesure du montage de mesure et pour produire une sortie signalant le message d'erreur de mouvement.

2. Appareil de mesure combiné selon la revendication 1, **caractérisé en ce qu'**un capteur d'accélération est connecté à la plate-forme et **en ce que** le dispositif d'enregistrement et de traitement de données (100) est organisé pour produire un message de soupçon signalant un mouvement possible du sujet si le signal du capteur d'accélération excède une première valeur de seuil d'accélération.

3. Appareil de mesure combiné selon la revendication 1 ou 2, **caractérisé en ce que** le dispositif d'enregistrement et de traitement de données (100) est organisé pour contrôler les résultats de mesure du montage de mesure pendant la période de mesure en fonction de critères de cohérence prédéfinis et, en cas de violation d'un critère de cohérence et de présence du message de soupçon de mouvement, pour rejeter la mesure du montage de mesure comme étant incorrecte et pour produire une sortie signalant le message de soupçon au sujet du mouvement du sujet.

4. Appareil de mesure combiné selon l'une des revendications précédentes 2 à 3, **caractérisé en ce que** le dispositif d'enregistrement et de traitement de données (100) est organisé, dans le cas où les signaux du capteur d'accélération excèderaient une seconde valeur de seuil d'accélération prédéfinie, qui est supérieure à la première, pour produire un message d'erreur signalant un mouvement du sujet.

5. Appareil de mesure combiné selon la revendication 4, **caractérisé en ce que** le dispositif d'enregistrement et de traitement de données (100) est organisé, en présence du message d'erreur de mouvement et après sortie du signalement à ce sujet, pour démarrer une nouvelle période de mesure pour le montage de mesure, afin de mesurer l'autre paramètre corporel.

6. Appareil de mesure combiné selon l'une des revendications précédentes, **caractérisé en ce qu'**une pluralité de cellules de pesage (WZ1, WZ2, WZ3, WZ4) est prévue et que le dispositif d'enregistrement et de traitement de données (100) est organisé pour enregistrer séparément, en plus de la somme des signaux de mesure des cellules de pesage, un résultat de mesure de poids de cellule de pesage pour chaque cellule de pesage et contrôler, pendant la période de mesure, pour chaque cellule de pesage, l'écart du signal de mesure par rapport au résultat de mesure de poids de cellule de pesage et, dans le cas où l'écart excéderait un seuil prédéfini pour une cellule de pesage individuelle, produire le message d'erreur signalant un mouvement du sujet.

7. Appareil de mesure combiné selon la revendication 6, **caractérisé en ce que** le dispositif d'enregistrement et de traitement de données (100) est organisé pour établir les différences entre des résultats de mesure de poids de cellule de pesage des cellules de pesage individuelles et, dans le cas où une différence excéderait une valeur de seuil de différence prédéfinie, pour produire un message de soupçon de positionnement erroné signalant un positionnement erroné du sujet, afin d'être utilisé lors du contrôle de la fiabilité du résultat de mesure pour l'autre paramètre corporel.

8. Appareil de mesure combiné selon la revendication 7, **caractérisé en ce que** le dispositif d'enregistrement et de traitement de données (100) est organisé pour contrôler les résultats de mesure du montage de mesure pendant la période de mesure en fonction de critères de cohérence prédéfinis et, en cas de violation d'un critère de cohérence et de présence du message de soupçon de positionnement erroné, pour rejeter la mesure du montage de mesure comme étant incorrecte, pour produire une sortie à ce sujet, et pour produire une sortie signalant le message de soupçon au sujet du positionnement erroné du sujet.
